# DEMANDE DE BREVET EUROPEEN

(11) **EP 4 424 300 A1**
(43) Date de publication de la demande: **04.09.2024**
(21) Numéro de dépôt: 24160688.8
(22) Date de dépôt: 29.02.2024
(51) Int. Cl.: A61K 8/99, A61Q 19/00

(54) **EXTRAIT DE SPHINGOMONAS PANACITERRAE ET SES UTILISATIONS COSMÉTIQUES**

(30) Priorité: 01.03.2023 FR 2301915
(71) Demandeur: Societe Industrielle Limousine d'Application Biologique, 19130 Objat (FR)
(72) Inventeur: PAUFIQUE, Jean, 19130 OBJAT (FR)
(74) Mandataire: Aquinov

(57) **Abrégé**

La présente invention concerne un principe actif cosmétique obtenu à partir d'une bactérie de l'espèce *Sphingomonas panaciterrae* et ses utilisations cosmétiques, en particulier pour accélérer le métabolisme épidermique, renforcer la fonction barrière de la peau, améliorer l'hydratation cutanée et raviver l'éclat du teint.

## Description

### Domaine technique

L'invention concerne un principe actif cosmétique obtenu à partir d'une bactérie de l'espèce *Sphingomonas panaciterrae* et ses utilisations cosmétiques, notamment pour renforcer la fonction barrière de la peau, améliorer l'hydratation cutanée et raviver l'éclat du teint.

### Etat de l'art

La vitalité est associée à un sentiment de bonne santé ou encore de jeunesse. Des peaux en manque de vitalité donnent un air fatigué susceptible d'accentuer les signes de l'âge et peuvent également traduire une mauvaise hygiène de vie. Ainsi, une peau manquant de vitalité peut avoir un impact important sur l'estime de soi et l'image que l'on renvoie.

Dans un quotidien de plus en plus effréné, les attentes des consommateurs concernant le maintien d'une vitalité optimale se font de plus en plus omniprésentes.

Il existe donc un besoin pour de nouveaux produits cosmétiques pour renforcer la fonction barrière de la peau, améliorer l'hydratation de la peau, accélérer le renouvellement cellulaire, raviver l'éclat du teint, et combler le manque de vitalité auquel la peau peut être confrontée.

Pour répondre à ce besoin, l'inventeur s'est intéressé à des matières premières naturelles pour développer un nouveau principe actif cosmétique, à savoir la biomasse des bactéries. Celle-ci représente environ 15% de la biomasse terrestre, après la flore végétal (80%). Bien que moins représentée, la biomasse bactérienne est indispensable au développement de la vie sur Terre. En effet, les micro-organismes rythment le développement des végétaux, de la graine, en passant par la plante, jusqu'à l'arbre. Ceux-ci agissent en particulier au niveau de la rhizosphère, lieu d'échange comprenant une très grande diversité bactérienne. Aussi, qu'ils soient néfastes, neutres ou bénéfiques, les microorganismes racinaires peuvent impacter la vitalité de la plante.

La croissance et la santé des végétaux sont notamment guidés par les rhizobactéries qui favorisent la croissance des plantes (également appelées PGPR - *Plant Growth-Promoting Rhizobacteria).*

Dans ce contexte, l'inventeur s'est particulièrement intéressé à une bactérie de la rhizosphère du ginseng. Cette plante est connue pour être une plante auto-toxique, qui appauvrit considérablement les sols et entraîne un stress environnemental important. Or, c'est au sein de cet environnement peu favorable, que la bactérie *Sphingomonas panaciterrae* a été identifiée.

Pour survivre avec peu d'éléments nutritifs et résister aux molécules toxiques, *Sphingomonas panaciterrae* a mis en place des mécanismes d'adaptation et de résistance. Dans cet environnement hostile, elle est également capable d'entrer en compétition avec les microorganismes pathogènes, d'améliorer la biodisponibilité des nutriments et de synthétiser des métabolites bénéfiques, ce qui confèrent à cette bactérie spécifique des propriétés remarquables, ainsi qu'un « capital vitalité ». Or, quel que soit l'organisme vivant considéré, la vitalité représente un puissant gage de bonne santé.

Ainsi, l'inventeur s'est particulièrement intéressé à la bactérie de l'espèce *Sphingomonas panaciterrae* pour développer un nouvel ingrédient dérivé du naturel. Il est déjà décrit des effets cosmétiques concernant des produits à base de *Sphingomonas sp.,* mais aucun à base d'extrait de *Sphingomonas panaciterrae.*

### Résumé de l'invention

Ainsi, l'invention concerne un extrait de *Sphingomonas panaciterrae,* en particulier un principe actif cosmétique comprenant ledit extrait de *Sphingomonas panaciterrae.*

Préférentiellement, l'extrait selon l'invention comprend des exopolysaccharides, à savoir des oligosphinganes, plus particulièrement des oligosphinganes d'une masse moléculaire inférieure à 9 kDa, encore plus préférentiellement des oligosphinganes ayant une masse moléculaire comprise entre 2,7 et 4,5 kDa soit de degré de polymérisation (DP) compris entre 15 et 25.

Selon un objet préféré de l'invention, l'extrait est obtenu à partir de la culture de *Sphingomonas panaciterrae,* comprenant notamment la bactérie, le milieu de culture et tout autre élément susceptible d'être présent dans le dispositif de culture, par exemple les molécules sécrétées lors de la culture. Avantageusement, la culture des bactéries *Sphingomonas panaciterrae* est carencée en azote.

Afin d'optimiser l'efficacité du principe actif cosmétique comprenant l'extrait de *Sphingomonas panaciterrae* selon l'invention, ledit extrait est préférentiellement obtenu par une hydrolyse, plus préférentiellement une hydrolyse enzymatique. L'extrait peut être obtenu par tout moyen, avantageusement, celui-ci est obtenu par deux hydrolyses successives, à savoir une hydrolyse des protéines et une hydrolyse des sucres.

Enfin, de façon particulièrement avantageuse, le principe actif cosmétique selon l'invention est susceptible d'être obtenu par un procédé d'extraction comprenant les étapes suivantes :
a. Culture de *Spingomonas panaciterrae* dans un milieu de culture adapté à la culture des bactéries et carencé en azote,
b. Solubilisation de la culture de *Sphingomonas panaciterrae,* dans l'eau a raison d'au moins 20 g/L,
c. Extraction par hydrolyse,
d. Traitement thermique,
e. Séparation des phases soluble et insoluble par décantation ou centrifugation et/ou filtration, et récupération de la phase soluble,
f. Eventuellement, tri moléculaire,
g. Éventuellement, désodorisation et/ou décoloration,
h. Éventuellement, filtration et filtration stérilisante

Selon un autre aspect, l'invention se rapporte également au procédé d'extraction et de préparation décrit précédemment.

Selon un autre objet préféré, le principe actif cosmétique comprenant l'extrait de *Sphingomonas panaciterrae* se présente sous forme liquide ou sous forme solide ou sous forme de film. Celui-ci comprend avantageusement au moins un excipient choisi parmi un conservateur, un antioxydant, un stabilisant, un support d'atomisation, et/ou leur combinaison.

Selon un autre aspect, l'invention se rapporte également à une composition cosmétique comprenant au moins 0,1% d'un principe actif cosmétique selon l'invention, en poids du poids total de la composition.

Selon un autre aspect, l'invention se rapporte plus particulièrement à l'utilisation du principe actif cosmétique selon l'invention ou de la composition le comprenant, destiné à un traitement cosmétique, en application topique sur la peau.

Préférentiellement, l'utilisation du principe actif cosmétique selon l'invention ou de la composition le comprenant, vise à renforcer la fonction barrière de la peau, et/ou améliorer l'hydratation de la peau.

L'utilisation du principe actif cosmétique selon l'invention ou de la composition le comprenant, vise également à accélérer le renouvellement cellulaire et/ou raviver l'éclat du teint.

Aussi, en rétablissant la vitalité cutanée, le principe actif selon l'invention relance le métabolisme épidermique, améliore la qualité de la barrière cutanée, les peaux jeunes et âgées retrouvent alors éclat et hydratation, permettant ainsi de répondre aux inconvénients de l'art antérieur.

D'autres caractéristiques et avantages ressortiront de la description détaillée de l'invention, des exemples et des figures qui vont suivre.

### Brève description des Figures

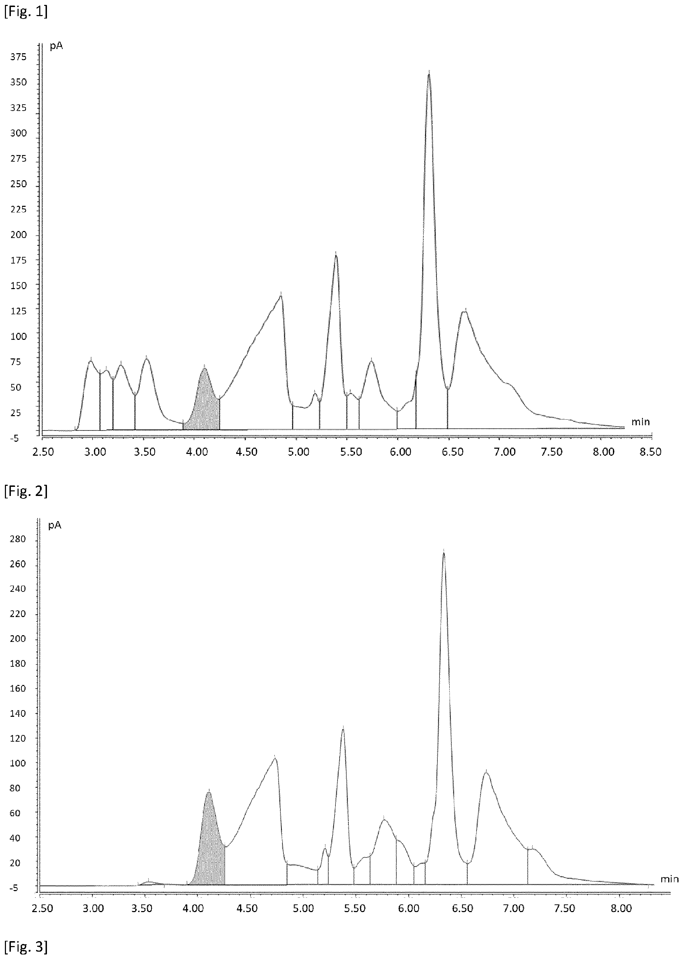

[Fig. 1] représente le profil chromatographique (LC-CAD - *Liquid Chromatography with Charged Aérosol Detection*) du principe actif selon l'exemple 1a. A partir de ce profil, l'aire du pic grisé, correspondant aux sucres de masse moléculaire entre 2 700 Da et 4 500 Da, soit un degré de polymérisation de DP15 à DP25, a été déterminé.
[Fig. 2] représente le profil chromatographique (LC-CAD - *Liquid Chromatography with Charged Aérosol Détection*) du principe actif selon l'exemple 1b. A partir de ce profil, l'aire du pic grisé, correspondant aux sucres de masse moléculaire entre 2 700 Da et 4 500 Da, soit un degré de polymérisation de DP15 à DP25, a été déterminé.
[Fig. 3] représente le profil chromatographique (LC-CAD - *Liquid Chromatography with Charged Aérosol Détection*) du principe actif selon l'exemple 1c. A partir de ce profil, l'aire du pic grisé, correspondant aux sucres de masse moléculaire entre 2 700 Da et 4 500 Da, soit un degré de polymérisation de DP15 à DP25, a été déterminé.

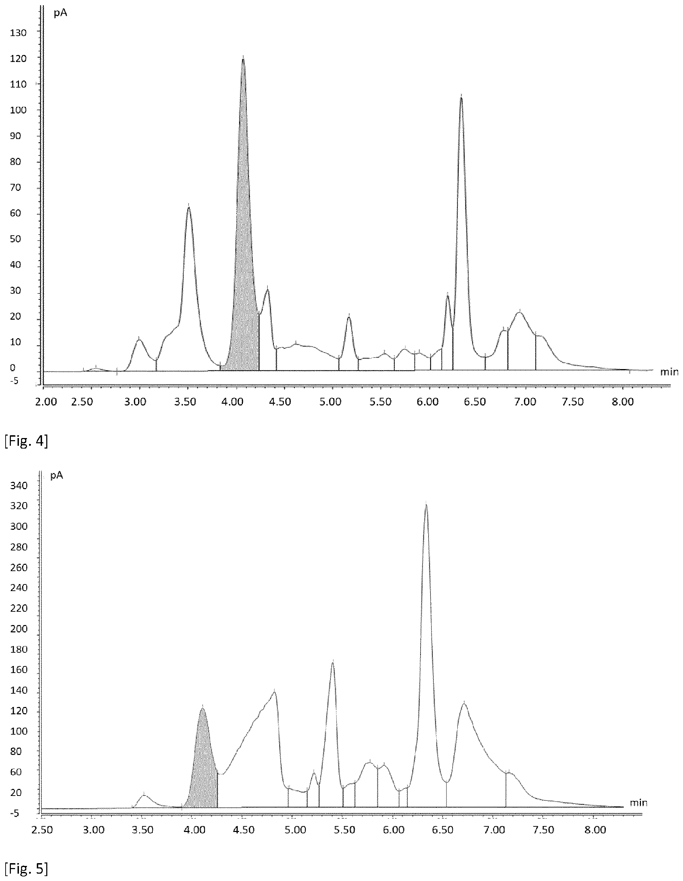

[Fig. 4] représente le profil chromatographique (LC-CAD - *Liquid Chromatography with Charged Aérosol Detection*) du principe actif selon l'exemple 2a. A partir de ce profil, l'aire du pic grisé, correspondant aux sucres de masse moléculaire entre 2 700 Da et 4 500 Da, soit un degré de polymérisation de DP15 à DP25, a été déterminé.
[Fig. 5] représente le profil chromatographique (LC-CAD - *Liquid Chromatography with Charged Aérosol Detection*) du principe actif selon l'exemple 2b. A partir de ce profil, l'aire du pic grisé, correspondant aux sucres de masse moléculaire entre 2 700 Da et 4 500 Da, soit un degré de polymérisation de DP15 à DP25, a été déterminé.

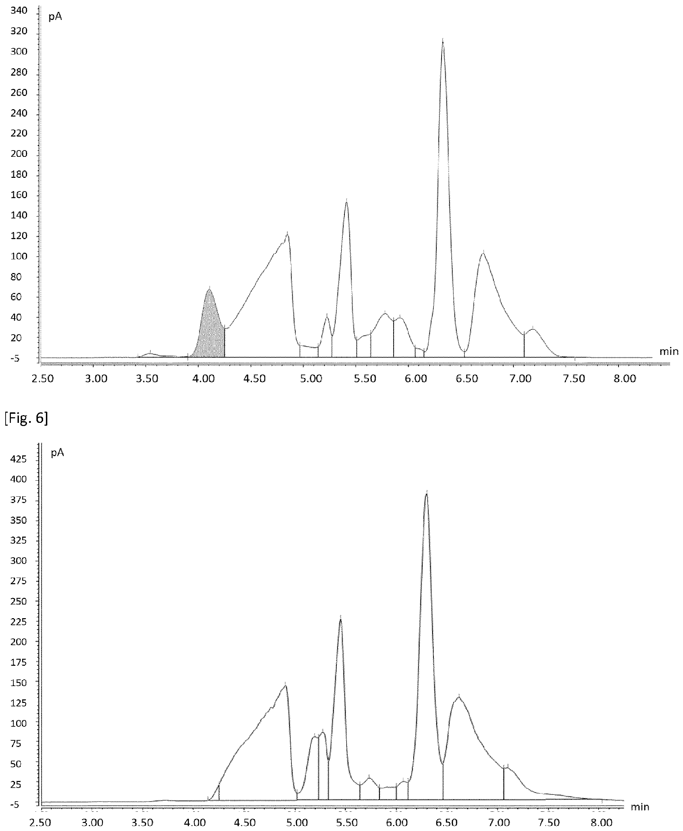

[Fig. 6] représente le profil chromatographique (LC-CAD - *Liquid Chromatography with Charged Aérosol Détection*) du principe actif selon l'exemple 3.

### Description détaillée de l'invention

### Définition

Par « extrait de *Sphingomonas panaciterrae* » au sens de la présente invention, on entend au moins une molécule ou toute molécule ou mélange d'au moins deux molécules obtenu(e) à partir d'une matière première, à savoir de la bactérie de l'espèce *Sphingomonas panaciterrae,* quel que soit le procédé d'extraction de ladite ou desdites molécule(s). Il peut s'agir par exemple d'un extrait obtenu par extraction aqueuse et/ou hydroalcoolique et/ou hydroglycolique et/ou obtenu après au moins une étape d'hydrolyse, par exemple enzymatique ou acide. Ainsi, au sens de l'invention un tel extrait est un ingrédient dérivé du naturel obtenu par des procédés chimiques et/ou biologiques spécifiques et définis visant à le modifier chimiquement, modifications qui sont intentionnelles, et qui permettent d'obtenir des structures de molécules qui ne sont donc plus identiques aux molécules présentes dans la nature.

Par « Extrait obtenu par une hydrolyse de *Sphingomonas panaciterrae* » au sens de l'invention, on entend un extrait issu de la bactérie de l'espèce *Sphingomonas panaciterrae,* obtenu par un procédé comprenant au moins une étape d'hydrolyse de *Sphingomonas panaciterrae.* Le terme extrait obtenu par hydrolyse de *Sphingomonas panaciterrae* exclut les molécules produites uniquement par fermentation de *Sphingomonas panaciterrae* par un micro-organisme ou une infusion/décoction/macération de *Sphingomonas panaciterrae.* Le terme extrait obtenu par hydrolyse exclut également les molécules obtenues par simple éclatement mécanique des cellules de *Sphingomonas panaciterrae.*

Par « principe actif cosmétique » au sens de l'invention, on entend un extrait comprenant au moins une molécule, préférentiellement un ensemble de molécules présentant un effet cosmétique sur la peau, lorsqu'il est appliqué topiquement.

### Principe actif selon l'invention

La présente invention a donc pour objet un principe actif cosmétique comprenant au moins un extrait de *Sphingomonas panaciterrae.*

La bactérie de l'espèce *Sphingomonas panaciterrae* appartient à la famille des *Sphingomonadaceae* regroupant une quinzaine de genres, dont *Sphingomonas. Sphingomonas panaciterrae* est une bactérie dite « rhizobactérie », favorisant la croissance des plantes, également appelée en anglais, *Plant Growth-Promoting Rhizobacteria (PGPR).* Ces rhizobactéries peuvent coloniser la rhizosphère et proliférer dans son environnement et possèdent un rôle complexe et crucial pour la pérennité de leur hôte. Fortes de ces caractéristiques, elles constituent aujourd'hui une alternative écologique à l'utilisation de produits chimiques dans la production agricole et la protection des cultures.

Les bactéries du genre *Sphingomonas* ont la particularité de sécréter des exopolysaccharides de types sphinganes tels que des gellan, welan, diutan etc. L'homme du métier spécialiste en bactérie sait que chaque souche de *Sphingomonas* produit des exopolysaccharides différents. A titre exemple, l'isolation de la souche *Sphingomonas* sp. CS101 a permis d'identifier un nouveau type de sphingane.

La bactérie *Sphingomonas panaciterrae* a ainsi été isolée dans le sol d'un champ de ginseng en Corée du Sud. Elle a la particularité de sécréter des exopolysaccharides (EPS) de type sphinganes, ce qui lui permet de se protéger et de protéger son hôte contre les stress environnementaux et ainsi survivre dans des environnements hostiles tels que la rhizosphère du ginseng.

L'accès (selon la définition du Protocole de Nagoya) à la *Sphingomonas panaciterrae* sous le numéro de souche KCTC 42346 a été effectué conformément à la règlementation nationale APA (Accès et Partage des Avantages) du pays d'origine (Corée du Sud).

Etant donné les propriétés remarquables de cette bactérie, l'inventeur a développé un principe actif comprenant l'extrait de *Sphingomonas panaciterrae* permettant de restaurer le capital vitalité, relancer le métabolisme épidermique et d'améliorer la qualité de la barrière cutanée sur des peaux jeunes et âgées, sublimant ainsi l'éclat du teint et restaurant l'hydratation cutanée.

L'inventeur a caractérisé les molécules constituant l'extrait selon l'invention et a notamment déterminé la teneur en sucres totaux et en protéines, respectivement selon la méthode de DUBOIS (Dubois M. et al., Analytical chemistry, 28, 3, 350-356, 1956), et la méthode de LOWRY (Lowry et al., Protein measurement with the folin reagent, J. Biol. Chem., 193, 265, 1951). La teneur en cendres minérales peut également être déterminée par la pesée des résidus issus de l'incinération à 550°C dans un four à moufle électrique.

Ainsi, le principe actif selon l'invention comprend au moins une fraction saccharidique, nommée sucres et une fraction protéique, nommée protéines.

Préférentiellement, l'extrait selon l'invention comprend au moins 15% de sucres totaux, au moins 15% de protéines en poids de la matière sèche de l'extrait. La teneur en cendres représente au moins 25% en poids de la matière sèche de l'extrait.

Parmi les sucres de l'extrait, l'extrait comprend majoritairement des oligosaccharides, plus particulièrement des oligosphinganes.

Ainsi, l'extrait de *Sphingomonas panaciterrae* comprend préférentiellement des oligosphinganes d'une masse moléculaire inférieure à 9 kDa, plus préférentiellement d'une masse moléculaire comprise entre 2,7 et 4,5 kDa. Par masse moléculaire comprise entre 2,7 et 4,5 kDa, on entend également un degré de polymérisation (DP) compris entre 15 et 25 soit des oligosphinganes de DP 15 à DP 25. Cette fraction d'oligosphinganes confère notamment au principe actif selon l'invention une capacité accrue à restaurer l'expression d'au moins un gène traceur de vitalité cutanée, à savoir le gène MAP3K1.

L'extrait comprend également des protéines, plus particulièrement des peptides de masse moléculaire inférieure à 3500 Da.

L'inventeur a également caractérisé et quantifié les minéraux présents dans l'extrait selon l'invention. Préférentiellement, l'extrait comprend au moins du chlore, du soufre, du sodium, du potassium, du magnésium, du phosphore et du calcium.

Selon un autre objet préféré de l'invention, l'extrait est un extrait de culture de *Sphingomonas panaciterrae,* c'est-à-dire un extrait obtenu à partir de la totalité de la culture de *Sphingomonas panaciterrae.* Plus préférentiellement, la culture des bactéries *Sphingomonas panaciterrae* est carencée en azote, permettant ainsi d'optimiser l'efficacité cosmétique du principe actif comprenant l'extrait.

Pour encore améliorer l'efficacité du principe actif, au moins une hydrolyse est mise en oeuvre. Ainsi, selon un objet particulièrement d'intérêt, l'extrait de *Sphingomonas panaciterrae* est issu d'une hydrolyse, plus préférentiellement une hydrolyse enzymatique.

Dans l'objectif d'optimiser le principe actif cosmétique et donc son efficacité cosmétique, l'extrait est très avantageusement obtenu au moyen de deux hydrolyses enzymatiques successives, à savoir une hydrolyse enzymatique à l'aide d'une protéase et une hydrolyse enzymatique à l'aide d'une carbohydrase.

Selon un objet particulièrement préféré de l'invention, l'extrait est susceptible d'être obtenu par un procédé de préparation comprenant les étapes suivantes :
a. Culture de *Spingomonas panaciterrae* dans un milieu de culture adapté à la culture des bactéries et carencé en azote
b. Solubilisation de la culture de *Sphingomonas panaciterrae,* dans l'eau a raison d'au moins 20 g/L,
c. Extraction par hydrolyse,
d. Traitement thermique,
e. Séparation des phases soluble et insoluble, et récupération de la phase soluble,
f. Eventuellement, tri moléculaire,
g. Éventuellement, désodorisation et/ou décoloration,
h. Éventuellement, filtration et filtration stérilisante.

Le principe actif cosmétique selon l'invention comprend avantageusement au moins un excipient choisi parmi un conservateur, un antioxydant, un stabilisant, un support d'atomisation, et/ou leur combinaison.

L'extrait selon l'invention présent dans le principe actif cosmétique peut se présenter sous forme liquide ou sous forme solide ou sous forme de film.

Lorsqu'il se présente sous forme liquide, le principe actif selon l'invention est exclusivement constitué par l'extrait de *Sphingomonas panaciterrae* accompagné de stabilisant et/ou système de conservation.

L'extrait sous forme liquide se présente préférentiellement sous forme d'une solution aqueuse liquide limpide, avec une faible odeur et une couleur jaune très pâle à jaune clair. Il peut toutefois être plus coloré.

Lorsqu'il se présente sous forme liquide, la teneur en matières sèches de l'extrait peut être déterminée par la pesée des résidus issus du séchage de l'extrait selon l'invention à 105°C dans une étuve jusqu'à l'obtention d'un poids constant. Préférentiellement, l'extrait selon l'invention sous forme liquide a une teneur en matières sèches de 3g/L à 25g/L, préférentiellement de 7 g/L à 13 g/L.

Lorsqu'il se présente sous forme solide, le principe actif selon l'invention est préférentiellement constitué par l'extrait de *Sphingomonas panaciterrae* tel que précédemment décrit et par un support choisi parmi la maltodextrine, la gomme arabique ou la lécithine de soja. Selon un mode de réalisation, particulièrement adapté, l'extrait représente au moins 10% en poids du principe actif et le support au plus 90% en poids du principe actif.

Dans le cas d'une forme solide dans laquelle le principe actif est associé à un support, les teneurs en protéines, en sucres et en minéraux dans le principe actif sont modifiées, le support étant généralement constitué majoritairement de sucres.

Le principe actif selon l'invention peut aussi être présenté sous forme d'un film tel que décrit dans le brevet FR3079145. Dans ce cas, l'extrait de *Sphingomonas panaciterrae* représente préférentiellement au moins 0,1% en poids du film.

Lorsqu'il se présente sous forme de film, le principe actif comprend :
- au moins l'extrait de *Sphingomonas panaciterrae* selon l'invention.
- au moins une charge minérale, et
- au moins un polymère d'origine naturelle, et
- au moins un plastifiant, et
- au moins un tensio-actif.

Le polymère d'origine naturelle peut être choisi parmi la : Pectine, Gomme tamarin, Alginate, Pullulane, Psyllium, Xanthane, Guar, Tara, Caroube, Agar, Gomme arabique, Gellane, Dextran, Carraghénane, Cellulose, Konjac et le Chitosan.

Le plastifiant peut être choisi parmi : Glycérol, Sorbitol, Saccharose, Erythritol, Urée, Propylène glycol et le Butylène glycol.

La charge minérale peut être choisie parmi : Carbonate de calcium, argile verte, kaolin, perlite, talc, silicate de magnésium, mica, sericite diatomée, silice, sulfate de calcium, chlorure de calcium, chlorure de potassium, oxyde de fer et l'oxyde de zinc.

Le principe actif peut comprendre, en outre, un pigment pour colorer le film.

Composition cosmétique selon l'invention

Le principe actif selon l'invention peut éventuellement être intégré dans une composition cosmétique, notamment une composition comprenant au moins 0,1% en poids dudit principe actif et un milieu physiologiquement acceptable, préférentiellement un milieu cosmétiquement acceptable.

Ces compositions peuvent se présenter notamment sous forme d'émulsions huile-dans-eau, émulsions eau-dans-huile, émulsions multiples (Eau/Huile/Eau ou Huile/Eau/Huile) qui peuvent être éventuellement des microémulsions ou des nanoémulsions, ou sous forme de solutions, suspensions, hydrodispersions, gels aqueux, poudres, ou fond de teint ou sous forme de film. Elles peuvent être plus ou moins fluides et avoir l'aspect de crèmes, émulsions, gels, masques ou tous autres aspects des cosmétiques de soin de la peau saine.

Préférentiellement, il peut s'agir de compositions comprenant entre 0,1 et 20% du principe actif liquide selon l'invention, plus préférentiellement entre 1 et 10%.

Ces compositions comprennent, outre le principe actif, un milieu physiologiquement acceptable tel qu'un milieu cosmétiquement acceptable, c'est-à-dire qui ne provoque pas de sensations d'inconfort pour l'utilisateur telles que des rougeurs, tiraillements ou picotements.

Les compositions selon l'invention peuvent également contenir comme ingrédient au moins un composé choisi parmi :
- les huiles, qui peuvent être choisies notamment parmi les huiles de silicone, linéaires ou cycliques, volatiles ou non volatiles,
- les cires, telles que l'ozokérite, la cire de polyéthylène, la cire d'abeille ou la cire de carnauba,
- les élastomères de silicone,
- les tensioactifs, de préférence émulsionnants, qu'ils soient non ioniques, anioniques, cationiques ou amphotères,
- les co-tensioactifs, tels que les alcools gras linéaires,
- les épaississants et/ou gélifiants,
- les humectants, tels que les polyols comme la glycérine,
- les colorants, les conservateurs, les charges,
- les tenseurs,
- les séquestrants,
- les parfums,
- et leurs mélanges, sans que cette liste soit limitative.

Des exemples de tels ingrédients sont cités notamment dans le Dictionnaire CTFA (International Cosmetic ingrédient Dictionary and Handbook publié par le Personal Care Product Council).

Bien entendu, l'homme du métier veillera à choisir les éventuels composés complémentaires, actifs ou non-actifs, et leur quantité, de telle sorte que les propriétés avantageuses du mélange ne soient pas, ou sensiblement pas, altérées par l'adjonction envisagée.

Procédé de préparation de l'extrait selon l'invention

L'extrait constituant ou contenu dans le principe actif selon l'invention peut être obtenu par tout moyen.

Préalablement au procédé d'obtention de l'extrait en tant que tel, une culture de la biomasse bactérienne de *Sphingomonas panaciterrae* est réalisée dans un milieu adapté à leur développement, plus particulièrement un milieu de culture adapté, de manière classique pour l'homme de métier mais carencé en azote. Une fois la culture obtenue, on réalise une étape d'extraction, destinée à extraire les molécules d'intérêt. Très préférentiellement, l'extraction comprend au moins une hydrolyse enzymatique en vue d'obtenir les molécules actives.

Ainsi, selon un mode de réalisation préféré, le procédé d'extraction comprend au moins une étape d'extraction de la bactérie *Sphingomonas panaciterrae,* tel qu'au moins une hydrolyse, préférentiellement au moins une hydrolyse enzymatique. Plus préférentiellement l'étape d'extraction comprend deux hydrolyses, encore plus préférentiellement deux hydrolyses enzymatiques afin d'optimiser l'extraction des molécules d'intérêt, notamment les sucres et les protéines d'intérêt.

Selon un mode de réalisation particulièrement adapté, le principe actif selon l'invention est obtenu par la mise en oeuvre des étapes suivantes :
a. Culture de *Sphingomonas panaciterrae* dans un milieu de culture adapté à la culture des bactéries et carencé en azote,
b. Solubilisation de la culture de *Sphingomonas panaciterrae,* dans l'eau à raison d'au moins 20 g/L,
c. Extraction par hydrolyse,
d. Traitement thermique,
e. Séparation des phases soluble et insoluble, et récupération de la phase soluble,
f. Eventuellement, tri moléculaire,
g. Éventuellement, désodorisation et/ou décoloration,
h. Éventuellement, filtration et filtration stérilisante.

L'étape de traitement thermique est avantageusement réalisée pour inactiver la ou les enzyme(s) présente(s). Cette inactivation est alors réalisée selon les préconisations du fournisseur de la ou les enzyme(s) utilisée(s).

La séparation de la phase soluble et insoluble est réalisée par tout moyen connu de l'homme du métier, par exemple par centrifugation, filtration ou décantation. La séparation des phases soluble et insoluble est réalisée pour récupérer la phase soluble.

L'étape de tri moléculaire permet la purification de la phase soluble récupérée. Elle est réalisée afin de sélectionner plus spécifiquement les molécules actives.

L'extrait obtenu à ce stade peut éventuellement être encore concentré et/ou purifié, préférentiellement par des étapes d'ultrafiltration successives à travers des membranes de porosité différentes, en conservant les molécules actives à chaque étape et/ou par une méthode de type chromatographique.

L'extrait obtenu après hydrolyse et filtration, avant ou après concentration et filtration stérilisante, est un extrait de *Sphingomonas panaciterrae,* et constitue une première forme du principe actif selon l'invention, se présentant alors sous forme liquide.

Ainsi, l'extrait obtenu peut ensuite être séché et associé ou non à un support, pour se présenter sous forme solide. Cette phase peut être réalisée par la mise en oeuvre des étapes suivantes :
- un support d'atomisation, de préférence la maltodextrine, est ajouté dans l'extrait de *Sphingomonas panaciterrae,* d'au plus 90% (en masse/volume) ;
- cette solution est ensuite concentrée sous vide ;
- un traitement antimicrobien peut être réalisé ;
- l'atomisation permet d'obtenir une poudre.

Les étapes des procédés décrits ci-avant, prises individuellement sont usuelles dans le domaine des extractions d'actifs à partir de matières premières naturelles et l'homme du métier est à même d'en ajuster les paramètres réactionnels sur la base de ses connaissances générales.

### Utilisation cosmétique

Le principe actif selon l'invention ou la composition selon l'invention est destiné à être utilisé sur des peaux saines, notamment des peaux saines manquant de vitalité.

Ainsi, selon un objet, l'invention se rapporte à l'utilisation du principe actif cosmétique selon l'un des quelconques modes de réalisation précédemment décrit ou d'une composition le comprenant, destiné à un traitement cosmétique en application topique sur la peau.

Préférentiellement l'utilisation selon l'invention se rapporte à accélérer le renouvellement cellulaire, et/ou raviver l'éclat du teint.

Selon un autre objet, l'utilisation vise également à renforcer la fonction barrière de la peau et/ou améliorer l'hydratation de la peau.

Ledit principe actif cosmétique selon l'invention agissant comme hydratant et boosteur d'éclat.

Préférentiellement, l'utilisation cosmétique du principe actif selon l'invention ou de la composition selon l'invention vise à activer les voies biologiques associées à la vitalité, en particulier en stimulant le métabolisme cellulaire ce qui se traduit par une augmentation de la production d'ATP et du renouvellement cellulaire ; ainsi qu'en renforçant la qualité de la barrière cutanée, ce qui se traduit par une diminution de la perte en eau et donc une amélioration de l'hydratation cutanée.

Aussi, l'éclat du teint est boosté et la peau retrouve son hydratation, que ce soit sur les peaux matures mais aussi sur les peaux jeunes.

L'invention est à présent illustrée par des exemples non limitatifs de composition selon l'invention et par des résultats.

### Exemples

### Exemple 1a : Principe actif selon l'invention (PA1a)

Le principe actif de l'exemple 1a est obtenu à partir d'une bactérie de l'espèce *Sphingomonas panaciterrae.* Le principe actif de l'exemple 1a est obtenu par le procédé suivant :
- Culture de *Sphingomonas panaciterrae* dans un milieu de culture adapté à la culture des bactéries et carencé en azote,
- Solubilisation de la culture de *Sphingomonas panaciterrae,* dans l'eau a raison d'au moins 20 g/L,
- Extraction par deux hydrolyses enzymatiques,
- Traitement thermique
- Séparation des phases soluble et insoluble, et récupération de la phase soluble,
- Filtration et filtration stérilisante.

Le principe actif selon l'exemple 1a est constitué d'oligosaccharides de masse moléculaire comprise entre 360 Da et 25 200 Da (DP 2 à DP 140). Il est notamment constitué d'une part d'oligosaccharides de masse moléculaire supérieure à 5 kDa.

Le profil chromatographique du principe actif selon l'exemple 1a présente un pic caractéristique correspondant à des oligosaccharides de DP 15 à 25 [Fig. 1]. L'aire du pic correspondant aux oligosphinganes de DP 15 à 25 représente 7,3% du total des aires du chromatogramme.

Le principe actif obtenu possède notamment les caractéristiques suivantes :
- Teneur en Matières Sèches = 18 g/L, dont :
- Teneur en Sucres Totaux = 6,3 g/L, soit 35%, en poids par rapport à la matière sèche
- Teneur en protéines = 4,7g/L, soit 26%, en poids par rapport à la matière sèche
- Teneur en cendres minérales = 5,6 g/L, soit 31% en poids par rapport à la matière sèche

### Exemple 1b : Principe actif selon l'invention (PA1b)

Le principe actif de l'exemple 1b est obtenu à partir d'une bactérie de l'espèce *Sphingomonas panaciterrae.* Le principe actif de l'exemple 1 est obtenu par le procédé suivant :
- Culture de *Sphingomonas panaciterrae* dans un milieu de culture adapté à la culture des bactéries et carencé en azote,
- Solubilisation de la culture de *Sphingomonas panaciterrae,* dans l'eau a raison d'au moins 20 g/L,
- Extraction par deux hydrolyses enzymatiques,
- Traitement thermique,
- Séparation des phases soluble et insoluble, et récupération de la phase soluble,
- Tri moléculaire,
- Filtration et filtration stérilisante.

Le principe actif selon l'exemple 1b est ainsi constitué majoritairement d'oligosaccharides de masse moléculaire comprise entre 360 Da et 9 000 Da (DP 2 à DP 50). Le profil chromatographique du principe actif selon l'exemple 1b, est représenté en [Fig. 2]. Il présente un pic caractéristique correspondant à la fraction active composée d'oligosaccharides de DP 15 à 25. L'aire du pic correspondant aux oligosphinganes de DP 15 à 25 représente 15% du total des aires du chromatogramme.

Le principe actif obtenu possède notamment les caractéristiques suivantes :
- Teneur en Matières Sèches = 10 g/L, dont :
- Teneur en Sucres Totaux = 2,7 g/L, soit 27%, en poids par rapport à la matière sèche
- Teneur en protéines = 1,6g/L, soit 16%, en poids par rapport à la matière sèche
- Teneur en cendres minérales = 4,5 g/L, soit 45% en poids par rapport à la matière sèche

### Exemple 1c : Principe actif selon l'invention (PA1c)

Le principe actif de l'exemple 1c est obtenu à partir d'une bactérie de l'espèce *Sphingomonas panaciterrae.* Le principe actif de l'exemple 1c est obtenu par le procédé suivant :
- Culture de *Sphingomonas panaciterrae* dans un milieu de culture adapté à la culture des bactéries et carencé en azote,
- Solubilisation de la culture de *Sphingomonas panaciterrae,* dans l'eau à raison d'au moins 20 g/L,
- Extraction avec deux hydrolyses enzymatiques,
- Traitement thermique,
- Séparation des phases soluble et insoluble, et récupération de la phase soluble,
- Tri moléculaire et récupération du rétentat pour éliminer les molécules de masses moléculaires inférieures à DP 15,
- Filtration et filtration stérilisante.

Le principe actif selon l'exemple 1c est constitué d'oligosaccharides de masse moléculaire supérieure à 2 700 Da (DP 15). Le profil chromatographique du principe actif selon l'exemple 1c présente un pic caractéristique correspondant à des oligosaccharides de DP 15 à 25 [Fig. 3]. L'aire du pic correspondant aux oligosphinganes de DP 15 à 25 représente 36,4% par rapport au total des aires du chromatogramme.

Le principe actif obtenu possède notamment les caractéristiques suivantes :
- Teneur en Matières Sèches = 6,2 g/L, dont :
- Teneur en Sucres Totaux = 3,3 g/L, soit 53%, en poids par rapport à la matière sèche
- Teneur en protéines = 1,05 g/L, soit 17%, en poids par rapport à la matière sèche

### Exemple 2a : Principe actif selon l'invention (PA2a)

Le principe actif de l'exemple 2a est obtenu à partir d'une bactérie de l'espèce *Sphingomonas panaciterrae.* Le principe actif de l'exemple 2a est obtenu par le procédé suivant :
- Culture de *Sphingomonas panaciterrae* dans un milieu de culture adapté à la culture des bactéries et carencé en azote,
- Solubilisation de la culture de *Sphingomonas panaciterrae,* dans l'eau a raison d'au moins 20 g/L,
- Extraction avec une hydrolyse enzymatique à l'aide d'une protéase,
- Traitement thermique,
- Séparation des phases soluble et insoluble, et récupération de la phase soluble,
- Tri moléculaire,
- Filtration et filtration stérilisante.

Le principe actif selon l'exemple 2a est ainsi constitué majoritairement d'oligosaccharides de masse moléculaire comprise entre 360 Da et 9 000 Da (DP 2 à DP 50). Le profil chromatographique obtenu présente un pic caractéristique des oligosaccharides de DP 15 à 25 [Fig. 4]. L'aire du pic correspondant aux oligosphinganes de DP 15 à 25 représente 16,6% par rapport au total des aires du chromatogramme.

Le principe actif obtenu possède notamment les caractéristiques suivantes :
- Teneur en Matières Sèches = 14,5 g/L, dont :
- Teneur en Sucres Totaux = 4,2 g/L, soit 29%, en poids par rapport à la matière sèche
- Teneur en protéines = 2,6 g/L, soit 18%, en poids par rapport à la matière sèche
- Teneur en cendres minérales = 5,9 g/L, soit 41% en poids par rapport à la matière sèche

### Exemple 2b : Principe actif selon l'invention (PA2b)

Le principe actif de l'exemple 2b est obtenu à partir d'une bactérie de l'espèce *Sphingomonas panaciterrae.* Le principe actif de l'exemple 2b est obtenu, sans hydrolyse enzymatique mais dans les mêmes conditions, par le procédé suivant :
- Culture de *Sphingomonas panaciterrae* dans un milieu de culture adapté à la culture des bactéries et carencé en azote,
- Solubilisation de la culture de *Sphingomonas panaciterrae,* dans l'eau à raison d'au moins 20 g/L,
- Traitement thermique
- Séparation des phases soluble et insoluble, et récupération de la phase soluble,
- Tri moléculaire,
- Filtration et filtration stérilisante.

Le principe actif selon l'exemple 2b est ainsi constitué majoritairement d'oligosaccharides de masse moléculaire comprise entre 360 Da et 9 000 Da (DP 2 à DP 50). Le profil chromatographique présente le pic caractéristique des oligosaccharides de DP 15 à 25 [Fig. 5]. L'aire du pic correspondant aux oligosphinganes DP 15-25 = 12,45, soit 11,9% par rapport au total des aires du chromatogramme.

Le principe actif obtenu possède notamment les caractéristiques suivantes :
- Teneur en Matières Sèches = 9,1 g/L, dont :
- Teneur en Sucres Totaux = 2,3 g/L, soit 25%, en poids par rapport à la matière sèche
- Teneur en protéines = 0,1 g/L, soit 1%, en poids par rapport à la matière sèche
- Teneur en cendres minérales = 4,8 g/L, soit 53% en poids par rapport à la matière sèche

### Exemple 3 : Extrait hors invention (HI1)

L'extrait de l'exemple 3 est obtenu selon le procédé décrit dans l'exemple 1b, à partir d'une bactérie de l'espèce *Sphingomonas kyeonggiensis,* bactérie isolée de la rhizosphère du ginseng. L'espèce *Sphingomonas kyeonggiensis* a été choisie parmi les autres bactéries du genre *Sphingomonas* car elle a été isolée dans la même rhizosphère que *Sphingomonas panaciterrae.*

L'extrait selon l'exemple 3 est constitué d'oligosaccharides de masse moléculaire comprise entre 360 Da et 2 700 Da (DP 2 à DP15). Le profil chromatographique de l'exemple 3 présente un pic correspondant à des oligosaccharides de DP 15 à 25 de faible intensité [Fig. 6]. L'aire du pic correspondant aux oligosphinganes DP 15-25 = 0,8, soit 0,7% par rapport au total des aires du chromatogramme.

L'extrait selon l'exemple 3 présente notamment les caractéristiques suivantes :
- Teneur en Matières Sèches = 14,4 g/L, dont :
- Teneur en Sucres Totaux = 0,9 g/L, soit 6%, en poids par rapport à la matière sèche
- Teneur en protéines = 3,1g/L, soit 21%, en poids par rapport à la matière sèche
- Teneur en cendres minérales = 6,4 g/L, soit 44% en poids par rapport à la matière sèche

Les caractéristiques de l'extrait selon l'exemple 3 ne permettent pas d'obtenir l'efficacité cosmétique recherchée.

### Exemple 4 : Composition selon l'invention

Un exemple de formulation comprenant le principe actif selon l'invention sous forme de gelée hydratante est présenté dans le tableau 1 suivant.

**[Tableau 1]**

| | **Ingrédients** | **%** |
|---|---|---|
| **A** | Eau Purifiée | qsp 100 |
| | Conservateur | qs |
| **B** | Xanthan Gum | 0,10 |
| | Isopentyldiol | 4,00 |
| | Pentylene Glycol | 4,00 |
| **C** | Polyacrylate Crosspolymer-11 | 1,00 |
| **D** | Glycerin & Glyceryl Polyacrylate | 5,00 |
| **E** | Trideceth-9 PG-Amodimethicone & Trideceth-12 | 2,00 |
| **F** | **Principe Actif Invention** | **2,50** |

La composition de l'exemple 4 peut notamment être obtenue par le procédé suivant :
- Ajouter B dans A sous agitation modérée, agiter jusqu'à homogénéité.
- Ajouter C puis D sous agitation modérée, agiter jusqu'à homogénéité.
- Ajouter successivement E et F.

La composition est alors sous forme d'un gel opalescent.

### Exemple 5 : Composition selon l'invention

Un exemple de formulation comprenant le principe actif selon l'invention sous forme d'une crème de nuit est présenté dans le tableau 2 suivant.

**[Tableau 2]**

| | **Ingrédients** | **%** |
|---|---|---|
| **A1** | Eau Purifiée | qsp 100 |
| | Conservateur | qs |
| | Butylène Glycol | 4,00 |
| **A2** | Glycérine | 3,00 |
| | Chondrus Crispus Extract | 0,25 |
| **B** | PEG-100 Stéarate & Glyceryl Stéarate | 3,00 |
| | Cetearyl Alcohol | 1,50 |
| | C10-18 Triglycerides | 2,00 |
| | Isopropyl Palmitate | 7,00 |
| | Caprylic/Capric Triglyceride | 5,00 |
| | Coco-Caprylate | 5,00 |
| | Dimethicone | 2,00 |
| **C** | Sodium Polyacrylate | 1,00 |
| **D** | **Principe Actif Invention** | **2,50** |

La composition de l'exemple 5 peut notamment être obtenue par le procédé suivant :
- Ajouter A2 dans A1 sous agitation modérée, agiter jusqu'à homogénéité et chauffer à 80°C.
- Placer B sous agitation et chauffer à 80°C.
- Emulsionner B dans A sous agitation cisaillante pendant 10 minutes.
- A partir de 40°C, sous agitation modérée, ajouter C puis D.

La composition est alors sous forme d'un émulsion épaisse, blanche et brillante.

### Exemple 6 : Composition selon l'invention

Un exemple de formulation comprenant le principe actif selon l'invention sous forme d'une crème hydratante pour le corps est présenté dans le tableau 3 suivant.

**[Tableau 3]**

| | **Ingrédients** | **%** |
|---|---|---|
| **A1** | Eau Purifiée | qsp 100 |
| | Conservateur | qs |
| | Butylène Glycol | 3,00 |
| **A2** | Glycérine | 2,00 |
| | Xanthan Gum | 0,20 |
| **B** | Pentaerythrityl Distearate | 1,00 |
| | Theobroma Grandiflorum Seed Butter & Tocopherol | 2,00 |
| | Cetearyl Alcohol | 1,00 |
| | Isononyl Isononanoate | 5,00 |
| | Diisopropyl Sebacate | 5,00 |
| | Dipentaerythrityl Hexacaprylate/Hexacaprate | 4,00 |
| | Hydroxyethyl Acrylate/Sodium Acryloyldimethyl Taurate Copolymer | 1,00 |
| **C** | **Principe Actif Invention** | **2,50** |

La composition de l'exemple 6 peut notamment être obtenue par le procédé suivant :
- Ajouter A2 dans A1 sous agitation modérée, agiter jusqu'à homogénéité et chauffer à 80°C.
- Placer B sous agitation et chauffer à 80°C.
- Emulsionner B dans A sous agitation cisaillante pendant 10 minutes.
- A température ambiante, sous agitation douce, ajouter C.

La composition est alors sous forme d'un gel crème souple, blanc et brillant.

### Exemple 7 : Composition selon l'invention

Un exemple de formulation comprenant le principe actif selon l'invention sous forme d'une crème énergisante est présenté dans le tableau 4 suivant.

**[Tableau 4]**

| | **Ingrédients** | **%** |
|---|---|---|
| **A1** | Eau Purifiée | qsp 100 |
| | Conservateur | qs |
| | Butylène Glycol | 3,00 |
| **A2** | Glycérine | 2,00 |
| | Acacia Senegal Gum & Xanthan Gum | 0,20 |
| **A3** | CI 77491 (Iron Oxides) & Phytic Acid & Sodium Hydroxide | 0,21 |
| | CI 77499 (Iron Oxides) & Phytic Acid & Sodium Hydroxide | 0,11 |
| | CI 77891 (Titanium Dioxide) & Phytic Acid & Sodium Hydroxide | 5,00 |
| | CI 77492 (Iron Oxides) & Phytic Acid & Sodium Hydroxide | 0,50 |
| **B** | Potassium Cetyl Phosphate | 3,00 |
| | Cetearyl Alcohol | 2,30 |
| | Hydrogenated Coconut Oil | 2,00 |
| | Cocoglycerides | 7,00 |
| | Diisopropyl Adipate | 5,00 |
| | Dicaprylyl Carbonate | 5,00 |
| | Dimethicone | 2,00 |
| | Dimethicone | 3,00 |
| | Hydroxyethyl Acrylate/Sodium Acryloyldimethyl Taurate Copolymer | 0,70 |
| **C** | **Principe Actif Invention** | **2,50** |

La composition de l'exemple 7 peut notamment être obtenue par le procédé suivant :
- Ajouter A2 dans A1 sous agitation modérée, agiter jusqu'à homogénéité.
- Ajouter A3 dans A1+A2 sous agitation modérée et chauffer à 80°C.
- Placer B sous agitation et chauffer à 80°C.
- Emulsionner B dans A sous agitation cisaillante pendant 4 minutes.
- A température ambiante, ajouter C sous agitation douce.

La composition est alors sous forme d'une émulsion souple et brillante, de couleur beige clair.

### Evaluation de l'efficacité du principe actif selon l'invention

Essai 1 - Evaluation *in vitro* de l'efficacité des différents principes actifs sur l'expression de gènes liés au métabolisme et à la protection des cellules cutanées.

L'objectif de cette étude est d'évaluer la capacité du principe actif selon l'invention et des principes actifs hors invention à moduler l'expression de gènes impliqués dans les voies biologiques de la vitalité cutanée, à savoir MAP3K1 (Mitogen-activated protein kinase kinase kinase 1) pour la voie du métabolisme ; et BAD (BCL2 associated agonist of cell death) pour la voie de la protection.

Le protocole est le suivant. D'une part, les kératinocytes humains carencés nutritionnellement (déplétion en facteurs de croissance) sont ensemencés et incubés à 37°C. Ensuite le milieu de culture est de nouveau éliminé et remplacé par du milieu carencé ou non en facteurs de croissance contenant les extraits testés à 0,5% (V/V). Les cellules sont récupérées et les ARN totaux extraits.

D'autre part, les kératinocytes humains carencés physiologiquement (vieillissement) sont ensemencés et incubés à 37°C. Puis, le milieu de culture est éliminé et remplacé par du milieu contenant les fractions testées à 0,5% (V/V). Les cellules sont récupérées et les ARN totaux extraits.

Les ARNs ont été analysés par la technique de PCR quantitative. Les ARNm des protéines témoins internes de référence, ont été analysés en parallèle des ARNm des marqueurs impliqués dans les différentes voies biologiques de la vitalité cutanée. Les résultats sont présentés dans le tableau 5 ci-après.

**[Tableau 5]**

| | **MÉTABOLISME** | | **PROTECTION** | |
|---|---|---|---|---|
| | **MAP3K1** (%) | **Efficacité / témoin carencé (%)** | **BAD (%)** | **Efficacité / témoin carencé (%)** |
| **Kératinocytes normaux** | | | | |
| Témoin | 100 | | 100 | |

| **Kératinocytes carencés nutritionnellement** | | | | |
|---|---|---|---|---|
| Témoin | 29 | | 30 | |
| Exemple 1a - Invention (PA1a) | 56 | 93 | 65 | 117 |
| Exemple 1b - Invention (PA1b) | 64 | 121 | 73 | 143 |
| Exemple 1c - Invention (PA1c) | 54 | 86 | 68 | 127 |
| Exemple 3 - Hors Invention (HI1) | 27 | 0 | 29 | 0 |

| **Kératinocytes carencés physiologiquement** | | | | |
|---|---|---|---|---|
| Témoin | 57 | | 59 | |
| Exemple 1a - Invention (PA1a) | 137 | 140 | 95 | 61 |
| Exemple 1b - Invention (PAlb) | 145 | 154 | 92 | 56 |
| Exemple 1c - Invention (PAlc) | 144 | 153 | 93 | 58 |
| Exemple 3 - Hors Invention (HI1) | 60 | 5 | 54 | 0 |

Testés à 0,5%, le principe actif selon l'invention (Exemple 1a, 1b, et 1c) régule significativement l'expression des gènes MAP3K1 et BAD dans des kératinocytes carencés nutritionnellement ou physiologiquement. A l'inverse, l'exemple Hors Invention (Exemple 3) ne régule pas l'expression de ces deux gènes dans des kératinocytes carencés nutritionnellement ou physiologiquement.

Ainsi, seuls les principes actifs selon l'invention présentent l'efficacité cosmétique recherchée, en particulier sur l'expression des gènes MAP3K1 et BAD dans des kératinocytes carencés nutritionnellement ou physiologiquement.

Essai 2 - Evaluation du principe actif selon l'invention sur le métabolisme énergétique.

L'objectif de cette étude est d'évaluer la capacité du principe actif selon l'invention à rétablir le métabolisme énergétique d'épidermes reconstitués carencés nutritionnellement (déplétés en facteurs de croissance) ou physiologiquement (vieillis) en suivant, par immunohistofluorescence, la synthèse d'aconitase, décrite comme le garde du corps de la mitochondrie.

Les épidermes carencés nutritionnellement sont préparés selon le protocole suivant. Les kératinocytes humains témoins sont ensemencés sur des inserts puis incubés à 37°C. Puis, le milieu de culture est éliminé et remplacé par du milieu carencé ou non en facteurs de croissance contenant le principe actif selon l'invention à 0,1% ou 0,5% (V/V). Finalement, les épidermes sont récupérés, fixés, déshydratés et inclus en paraffine puis des coupes (4 µm) sont ensuite réalisées.

Les épidermes carencés physiologiquement sont préparés selon le protocole suivant. Les kératinocytes humains sont cultivés et répliqués dans un milieu de culture adapté.

Les kératinocytes humains témoins ou vieillis sont ensemencés sur des inserts puis incubés à 37°C. Puis, les épidermes sont traités ou non en systémique avec le principe actif selon l'invention à 0,1% et 0,5% (V/V). Enfin, les épidermes sont récupérés, fixés, déshydratés et inclus en paraffine. Des coupes (4 µm) sont ensuite réalisées.

Les résultats sont présentés dans le tableau 6 ci-après.

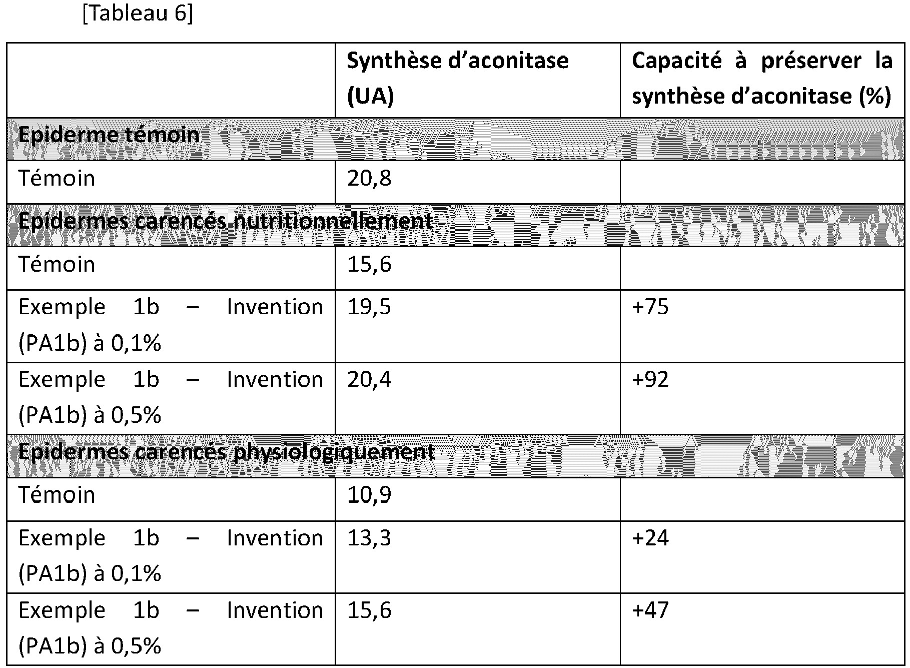

Les épidermes carencés nutritionnellement ou physiologiquement présentent une altération du métabolisme énergétique caractérisée par une diminution significative de la synthèse d'aconitase.

Testé à 0,5% sur des épidermes carencés, le principe actif selon l'invention préserve significativement la synthèse d'aconitase de 92% dans le cas d'une carence nutritionnelle, et restaure significativement la synthèse d'aconitase de 47% dans le cas d'une carence physiologique. Par conséquent, en stimulant le fonctionnement mitochondrial, le principe actif selon l'invention permet de rétablir le métabolisme énergétique d'épidermes carencés.

Essai 3 - Evaluation du principe actif selon l'invention sur les mécanismes de protection.

L'objectif de cette étude est d'évaluer la capacité du principe actif selon l'invention à renforcer les mécanismes de protection (anti-oxydante et mitochondriale) d'épidermes carencés nutritionnellement (déplétés en facteurs de croissance) ou physiologiquement (vieillis), en suivant, par immunohistofluorescence, la synthèse de la catalase, une enzyme antioxydante cruciale.

Le protocole est identique à celui précédemment décrit (Essai 2).

Les résultats sont décrits dans les tableaux 7 et 8 ci-après.

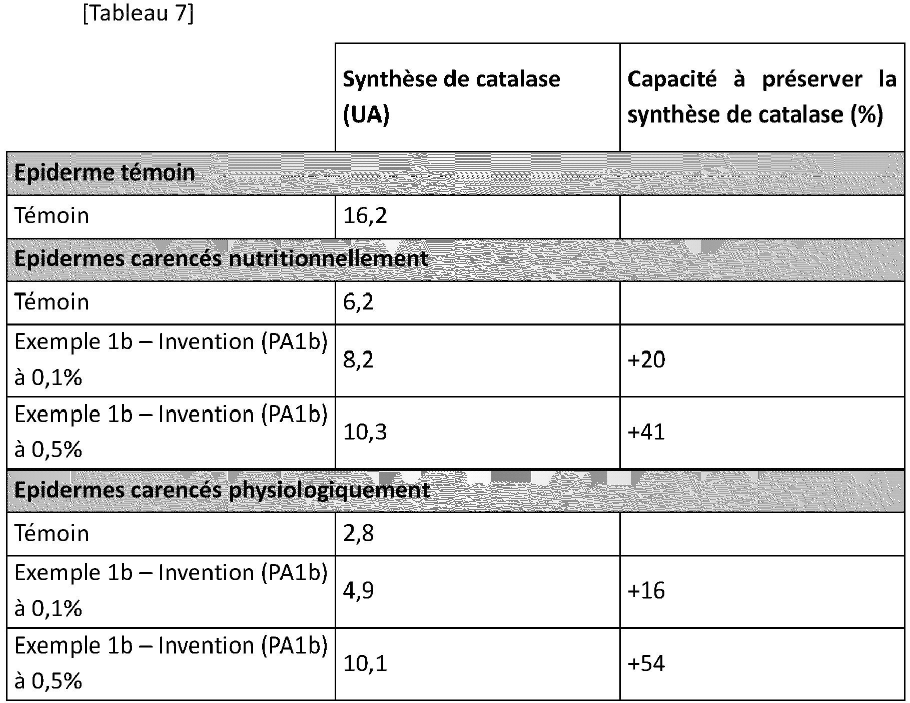

Les épidermes carencés nutritionnellement ou physiologiquement présentent une altération de leurs systèmes de protection.

Testé à 0,5% sur des épidermes carencés, le principe actif selon l'invention préserve significativement la synthèse de catalase de 41% dans le cas d'une carence nutritionnelle, et restaure significativement la synthèse de catalase de 54% dans le cas d'une carence physiologique. Le principe actif selon l'invention permet ainsi de renforcer les mécanismes de protection d'épidermes carencés.

Essai 4 - Evaluation du principe actif selon l'invention sur la fonction de la barrière cutanée.

L'objectif de cette étude est d'évaluer la capacité du principe actif selon l'invention à renforcer la barrière cutanée d'épidermes carencés nutritionnellement (déplétés en facteurs de croissance) ou physiologiquement (vieillis). En effet, une stratégie majeure de la peau pour se protéger est de maintenir une barrière intacte. Cette barrière résulte de la structure particulière du *stratum corneum* obtenue grâce aux processus de prolifération et de différenciation, et de la cohésion des couches supérieures de l'épiderme assurée par les jonctions serrées.

La reconstruction épidermique a été évaluée à l'aide d'une coloration hématoxyline éosine (HE) permettant de déterminer l'épaisseur des couches vivantes des épidermes. Enfin, l'intégrité des jonctions serrées a été analysée par la mesure de la résistance électrique transépithéliale (TEER).

Le protocole est identique à celui précédemment décrit (Essai 2).

Les résultats sont décrits dans les tableaux 9 et 10 ci-après.

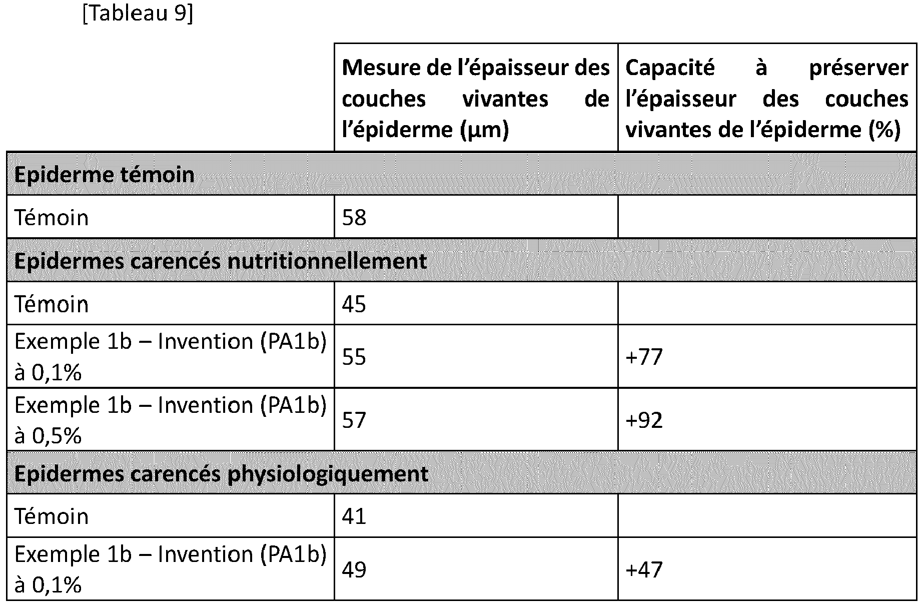

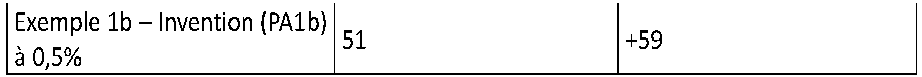

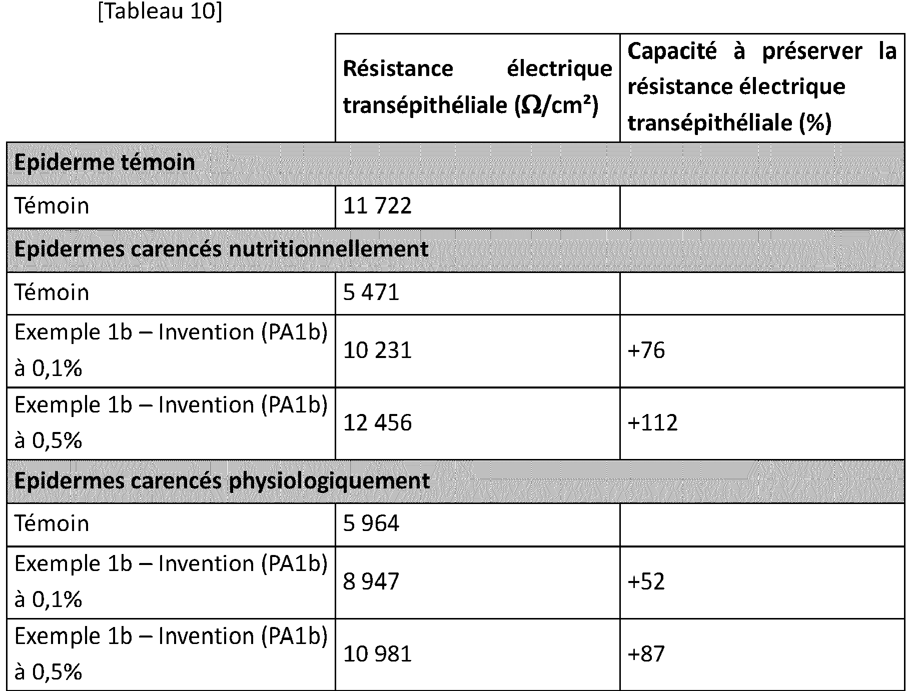

Les épidermes carencés nutritionnellement ou physiologiquement présentent une altération de leur fonction barrière, caractérisée par une diminution significative de l'épaisseur des couches vivantes de l'épiderme et de la résistance électrique transépithéliale.

Testé à 0,5% sur des épidermes carencés, le principe actif selon l'invention préserve significativement l'épaisseur des couches vivantes de l'épiderme de 92% et la résistance électrique transépithéliale de 112% dans le cas d'une carence nutritionnelle, et restaure significativement l'épaisseur des couches vivantes de l'épiderme de 59% et la résistance électrique transépithéliale de 87% dans le cas d'une carence physiologique. Aussi, en renforçant la fonction barrière cutanée, le principe actif selon l'invention permet ainsi de rétablir la vitalité cutanée d'épidermes.

Essai 5 - Evaluation *in vivo* des activités biologiques du principe actif selon l'invention.

L'objectif de cette étude est d'évaluer *in vivo,* en comparaison à une formule placebo, les activités biologiques du principe actif selon l'invention formulé à 2,5% en émulsion, chez des volontaires caucasiens jeunes et matures.

Pour y parvenir, la production d'ATP et de la qualité de la barrière cutanée au niveau du visage ont été étudiés, ainsi que le renouvellement cellulaire au niveau de la face interne des avant-bras.

Le protocole est le suivant.

La production d'ATP a été dosée par luminométrie et la qualité de la barrière cutanée a été déterminée par la mesure de la perte insensible en eau à l'aide d'un Téwamètre^{®} sur un panel jeune composé de 19 sujets sains, âgés de 26 à 40 ans (âge moyen 34 ± 5 ans), et un panel mature composé de 20 sujets sains, âgés de 50 à 69 ans (âge moyen 59 ± 7 ans). Les produits sont appliqués en hémi-visage de façon biquotidienne pendant 21 jours (panel jeune) ou 42 jours (panel mature).

Le renouvellement cellulaire au niveau de la face interne des avant-bras a été déterminé à l'aide du dermatoscope sur une zone préalablement définie et colorée à l'aide de la dihydroxyacétone (DHA) sur un panel jeune composé de 17 sujets sains, âgés de 27 à 40 ans (âge moyen 34 ± 4 ans), et un panel mature composé de 20 sujets sains, âgés de 50 à 71 ans (âge moyen 61 ± 7 ans). Les produits ont été appliqués de façon biquotidienne, sur la face interne des avant-bras sur une zone préalablement définie.

La fonction barrière a également été étudiée, après une agression au niveau de la face antéro-latérale externe des bras sur un panel jeune composé de 19 sujets sains, âgés de 26 à 40 ans (âge moyen 34 ± 5 ans). Les produits ont été appliqués de façon biquotidienne, pendant 14 jours

Les résultats sur la production d'ATP sont présentés dans le tableau ci-après.

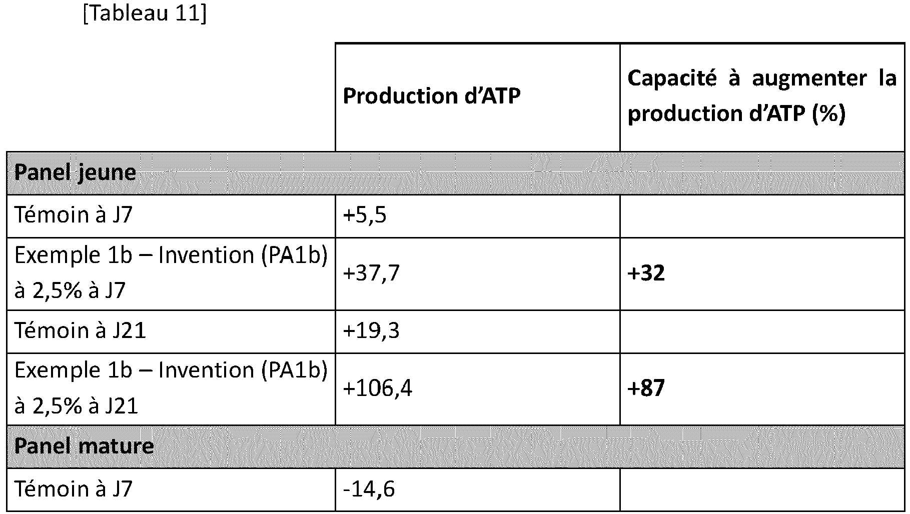

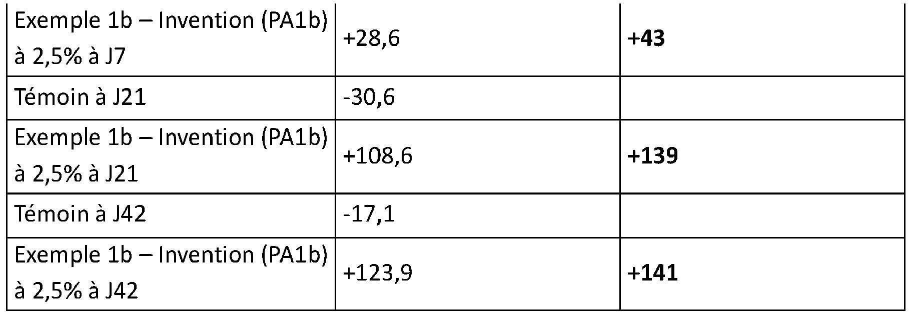

En comparaison au placebo, le principe actif selon l'invention stimule le métabolisme cellulaire.

En effet, dès 7 jours de traitement, le principe actif selon l'invention formulé à 2,5% en émulsion augmente la production d'ATP, respectivement, chez les volontaires caucasiens jeunes, de 32% et chez les volontaires caucasiens matures, de 43%. Ces effets s'intensifient encore après 21 jours de traitement chez 87% des volontaires jeunes et se prolongent jusqu'à 42 jours d'application chez les volontaires matures.

Les résultats sur le renouvellement épidermique sont présentés dans le tableau 12 ci-après.

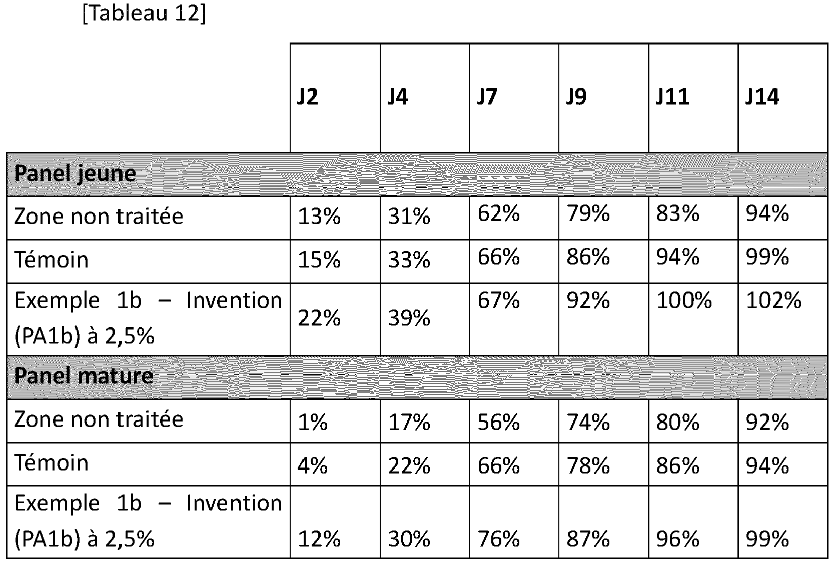

Après 2 jours, le principe actif selon l'invention formulé à 2,5% en émulsion favorise le renouvellement épidermique des volontaires caucasiens jeunes et matures, en augmentant significativement la capacité de leur peau à éliminer la DHA en comparaison au placebo.

Les résultats sur la fonction barrière cutanée sont présentés dans le tableau 13 ci-après.

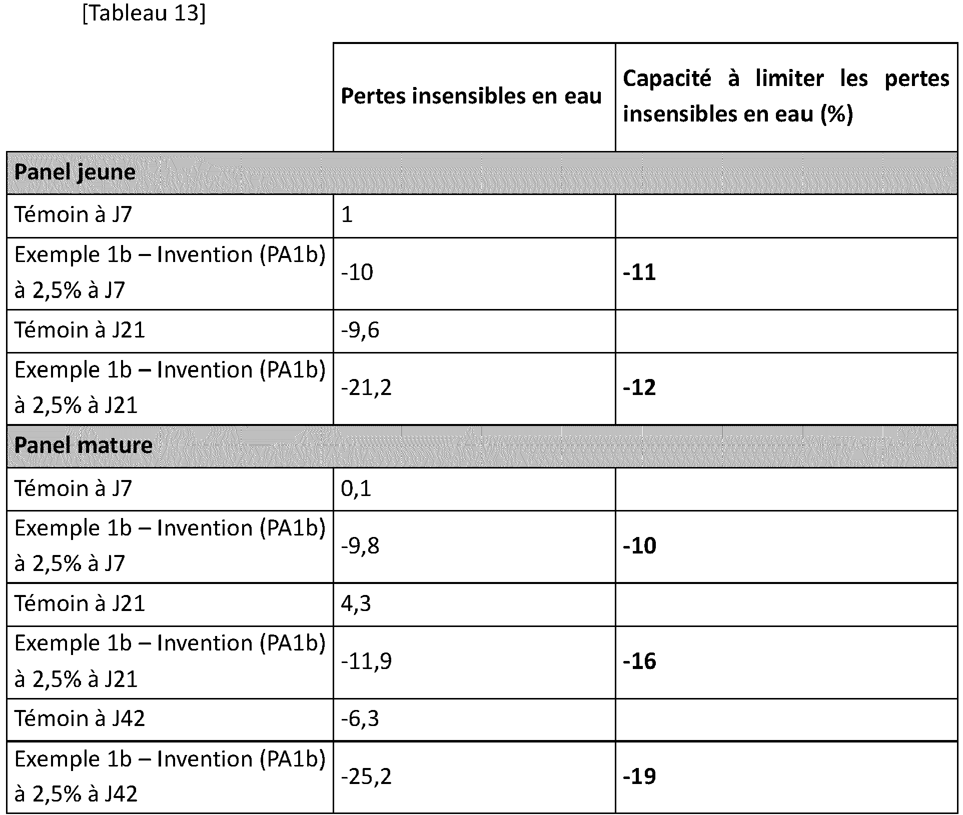

En comparaison au placebo, le principe actif selon l'invention améliore la qualité de la barrière cutanée. En effet, dès 7 jours, le principe actif selon l'invention formulé à 2,5% en émulsion diminue significativement la perte insensible en eau, chez les volontaires caucasiens jeunes de 11%, et chez les volontaires caucasiens matures de 10%. Cet effet se maintient après 21 jours chez les volontaires jeunes. Chez les volontaires matures, il s'intensifie après 21 jours et jusqu'à 42 jours d'application biquotidienne.

Les résultats sur la fonction barrière après agression sont présentés dans le tableau 14 ci-après.

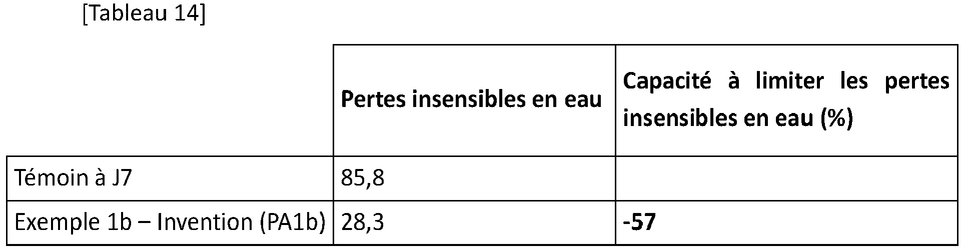

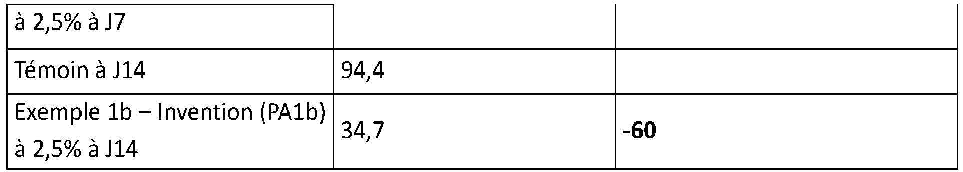

Dès 7 jours d'application et en comparaison au placebo, le principe actif selon l'invention formulé à 2,5% en émulsion protège la fonction barrière cutanée. En effet, le principe actif selon l'invention réduit significativement les pertes insensibles en eau des volontaires caucasiens jeunes après agression répétée au SLS de 57%. Cet effet s'accentue jusqu'à 14 jours d'application biquotidienne.

Par conséquent, le principe actif selon l'invention formulé à 2,5% en émulsion présente des effets rapides et significatifs sur la vitalité cutanée de volontaires caucasiens jeunes et matures. Dès 7 jours de traitement, le principe actif selon l'invention stimule le métabolisme et renforce la fonction barrière cutanée. Ces effets s'intensifient au cours du traitement.

Essai 6 - Efficacité cosmétique du principe actif selon l'invention

L'objectif de cette étude est d'évaluer in vivo, en comparaison à une formule placebo, les bénéfices cosmétiques du principe actif selon l'invention formulé à 2,5% en émulsion, chez des volontaires caucasiens jeunes et matures

Les panels jeune et mature sont identiques à ceux décrits dans l'essai 5 sur l'étude portant sur la production d'ATP. L'éclat du teint a été étudié par scorage visuel par experts et l'hydratation de la peau a été étudiée à l'aide d'un Cornéomètre^{®}. Les produits sont appliqués en hémi-visage de façon biquotidienne pendant 21 jours (panel jeune) ou 42 jours (panel mature).

Les résultats sur les principaux paramètres caractéristiques de l'éclat du teint sont présentés dans le tableau 15 ci-après.

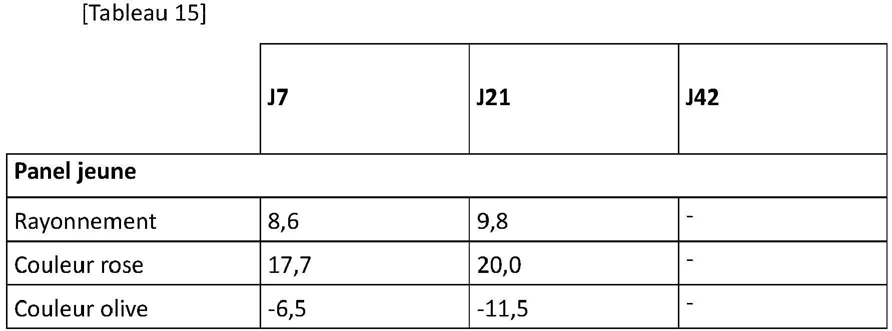

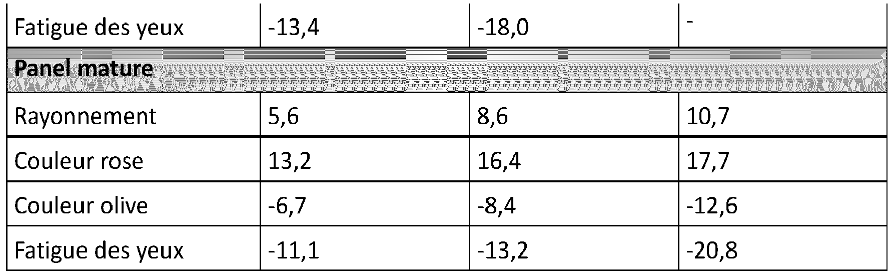

En comparaison au placebo, le principe actif selon l'invention améliore rapidement et de manière significative les paramètres caractéristiques de l'éclat du teint des peaux jeunes et matures caucasiennes. En effet, dès 7 jours de traitement, le principe actif selon l'invention formulé à 2,5% en émulsion rend le teint plus lumineux, plus frais et améliore l'aspect bonne mine en réduisant la couleur olive et la fatigue des yeux (effets significatifs par rapport au placebo). Ces effets s'intensifient après 21 jours de traitement chez les volontaires jeunes, avec une augmentation de 9,8% du rayonnement de la peau et de 20% de la couleur rose. L'inventeur observe également une diminution de la couleur olive de 11,5% et de l'état de fatigue des yeux de 18%.

Chez les volontaires matures, ces effets s'intensifient jusqu'à 42 jours de traitement, avec une augmentation de 10,7% du rayonnement de la peau et de 17,7% de la couleur rose. L'inventeur observe également une diminution de la couleur olive de 12,6% et de l'état de fatigue des yeux de 20,8%.

Les résultats sur l'hydratation cutanée sont présentés dans le tableau 16 ci-après.

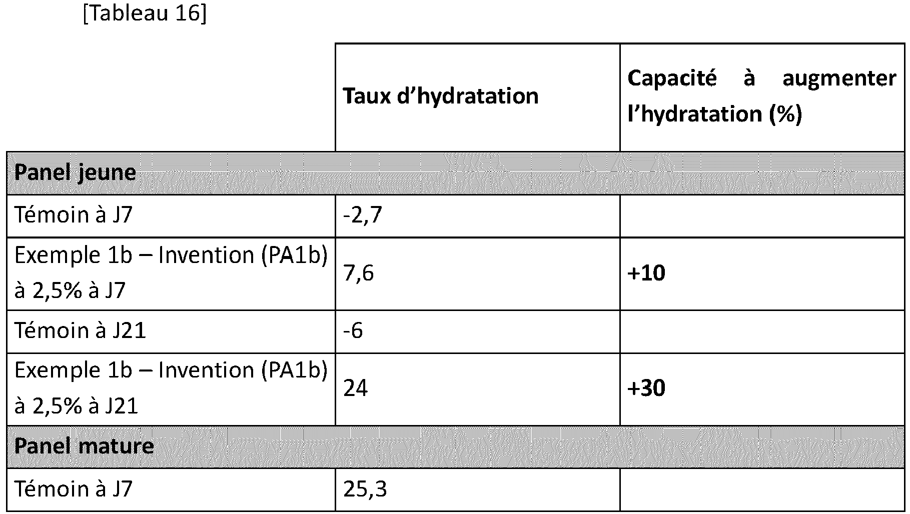

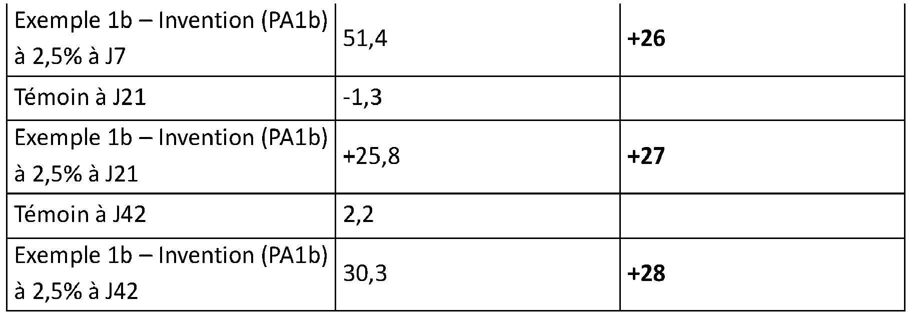

En comparaison au placebo, le principe actif selon l'invention améliore rapidement l'hydratation des peaux jeunes et matures caucasiennes. En effet, dès 7 jours de traitement biquotidien, le principe actif selon l'invention formulé à 2,5% en émulsion augmente l'hydratation de la peau du visage chez les volontaires jeunes, de 10%, et chez les volontaires matures, de 26%.

Cet effet s'intensifie après 21 jours de traitement chez le panel jeune, avec une augmentation significative de l'hydratation de 30%. Chez les volontaires matures, cet effet se maintient après 21 et 42 jours de traitement.

Par conséquent, dès 7 jours d'application biquotidienne, le principe actif selon l'invention formulé à 2,5% en émulsion procure des bénéfices cosmétiques aux peaux jeunes et matures caucasiennes en comparaison au placebo. Le principe actif selon l'invention sublime l'éclat du teint et améliore l'hydratation cutanée. Ces bénéfices cosmétiques ont également été perçus par les volontaires eux-mêmes.

## Revendications

1. Principe actif cosmétique comprenant au moins un extrait de *Sphingomonas panaciterrae.*

2. Principe actif cosmétique selon la revendication précédente, **caractérisé en ce que** ledit extrait comprend des oligosphinganes d'une taille inférieure à 9 kDa.

3. Principe actif cosmétique selon la revendication précédente, **caractérisé en ce que** les oligosphinganes ont une taille comprise entre 2,7 et 4,5 kDa.

4. Principe actif cosmétique selon l'une des revendications précédentes, **caractérisé en ce que** l'extrait est un extrait de culture de *Sphingomonas panaciterrae.*

5. Principe actif cosmétique selon l'une des revendications précédentes, **caractérisé en ce que** l'extrait est obtenu par une hydrolyse.

6. Principe actif cosmétique selon la revendication précédente, **caractérisé en ce que** l'extrait est obtenu par une hydrolyse enzymatique.

7. Principe actif cosmétique selon l'une des revendications précédentes, **caractérisé en ce que** ledit extrait est susceptible d'être obtenu par un procédé d'extraction comprenant les étapes suivantes :
a. Culture de *Sphingomonas panaciterrae* dans un milieu de culture adapté à la culture des bactéries et carencé en azote,
b. Solubilisation de la culture de *Sphingomonas panaciterrae,* dans l'eau a raison d'au moins 20 g/L,
c. Eventuellement, extraction par hydrolyse enzymatique,
d. Traitement thermique,
e. Séparation des phases soluble et insoluble, et récupération de la phase soluble,
f. Eventuellement tri moléculaire,
g. Éventuellement, désodorisation et/ou décoloration,
h. Éventuellement, filtration et filtration stérilisante.

8. Principe actif cosmétique selon l'une des précédentes revendications, **caractérisé en ce qu'**il se présente sous forme liquide ou sous forme solide ou sous forme de film.

9. Principe actif cosmétique selon l'une des précédentes revendications, **caractérisé en ce que** le principe actif comprend au moins un excipient choisi parmi un conservateur, un antioxydant, un stabilisant, un support d'atomisation, et/ou leur combinaison.

10. Utilisation d'un principe actif cosmétique selon l'une des revendications 1 à 9 ou d'une composition selon la revendication 15 destiné à un traitement cosmétique en application topique sur la peau.

11. Utilisation selon la revendication 10, pour renforcer la fonction barrière de la peau et/ou pour améliorer l'hydratation de la peau.

12. Utilisation l'une des revendications 10 ou 11, pour accélérer le renouvellement cellulaire.

13. Utilisation l'une des revendications 10 à 12, pour raviver l'éclat du teint.

14. Procédé de préparation d'un principe actif comprenant au moins un extrait de *Sphingomonas panaciterrae* selon l'une des revendication 1 à 9 comprenant les étapes suivantes :
a. Culture de *Sphingomonas panaciterrae* dans un milieu de culture adapté à la culture des bactéries et carencé en azote,
b. Solubilisation de la culture de *Sphingomonas panaciterrae,* dans l'eau a raison d'au moins 20 g/L,
c. Eventuellement, extraction par hydrolyse enzymatique,
d. Traitement thermique,
e. Séparation des phases soluble et insoluble, et récupération de la phase soluble,
f. Eventuellement, tri moléculaire,
g. Éventuellement, désodorisation et/ou décoloration,
h. Éventuellement, filtration et filtration stérilisante.

15. Composition cosmétique comprenant au moins 0,1% d'un principe actif cosmétique selon l'une des revendications 1 à 9, en poids du poids total de la composition.
